(19) Europäisches Patentamt
European Patent Office
Office européen des brevets

(11) Numéro de publication: **0 249 567 A2**

# (12) DEMANDE DE BREVET EUROPEEN

(21) Numéro de dépôt: 87420122.1

(22) Date de dépôt: 07.05.87

(51) Int. Cl.[3]: **C 07 C 43/20**
C 07 C 43/257, C 07 C 79/35-5
C 07 C 121/75, A 01 N 31/14
A 01 N 33/22, A 01 N 37/34

(30) Priorité: 12.05.86 FR 8606959

(43) Date de publication de la demande: 16.12.87 Bulletin 87/51

(84) Etats contractants désignés: AT BE CH DE ES FR GB GR IT LI LU NL SE

(71) Demandeur: RHONE-POULENC AGROCHIMIE
14-20, rue Pierre Baizet
F-69009 Lyon(FR)

(72) Inventeur: Place, Pierre
173, rue de la Mure Charly
F-69390 Vernaison(FR)

(72) Inventeur: Pépin, Régis
27, Montée Castellane
F-69140 Rillieux-la-Pape(FR)

(74) Mandataire: Chrétien, François et al,
RHONE-POULENC AGROCHIMIE BP 9163
F-69263 Lyon Cedex 09(FR)

(54) **Dérivés propargyloxybenzènes, leur préparation et compositions fongicides les contenant.**

(57) Nouveaux dérivés de propargyloxybenzène
Ils ont pour formule :

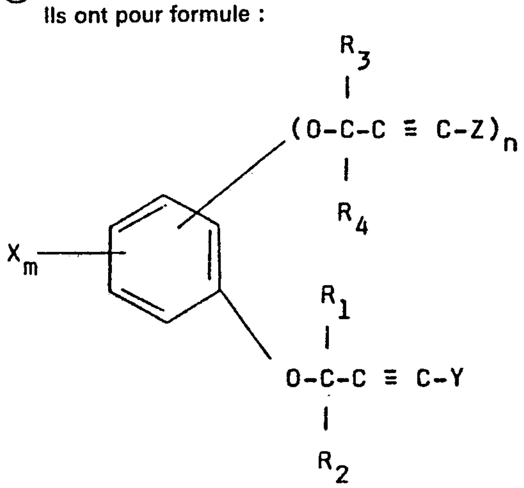

avec X = H, Hal, CN, $NO_2$, $NH_2$ éventuellement alcoylé, alcoyle ($C_1$-$C_{12}$), alcoxy ($C_1$-$C_{12}$), alcoylthio ($C_1$-$C_{12}$) éventuellement halogénés, alcoxylé ou benzoxylé ;

m = 0 à 5-n
Y,Z = H, Hal
n = 1,2
$R_1$ à $R_4$ = $H_1$, alcoyle inférieur

EP 0 249 567 A2

La présente invention a pour objet de nouveaux dérivés du propargyloxybenzène, leur préparation et leur utilisation comme matières actives de compositions agrochimiques en particulier fongicides.

L'utilisation répétée depuis des années d'un certain nombre de fongicides connus a amené l'apparition de souches de champignons phytopathogènes résistantes à ces fongicides. On rencontre ce phénomène avec la famille des benzimidazoles, notamment le carbendazime (benzimidazolyl-2) carbamate de méthyle, le bénomyl (butyl carbamoyl)-1 (benzimidazolyl-2) carbamate de méthyle, le thiophanate di [éthoxycarbonyl-3 thio-2 uréido]-1,2 benzène, le thiophanate méthyle di[méthoxycarbonyl-3-thio-2 uréido]-1,2 benzène ou encore avec la famille des imides cycliques notamment l'iprodione (dichloro-3,5 phényl)-3 isopropyl carbamoyl-1 imidazolidine dione-2,4, la procymidone (dichloro-3,5 phényl)-3 diméthyl-1,2 cyclopropane dicarboximide-1,2 et la vinchlozoline (dichloro-3,5 phényl)-3 méthyl-5 vinyl-5 oxazolidine dione-2,4. Ces résistances se rencontrent notamment sur Botrytis sp en particulier sur Botrytis cinerea.

L'invention a pour objet des produits fongicides actifs notamment sur des souches résistantes à des fongicides connus en particulier ceux de la famille des benzimidazoles.

Plus particulièrement l'invention concerne des dérivés du propargyloxybenzène de formule :

...

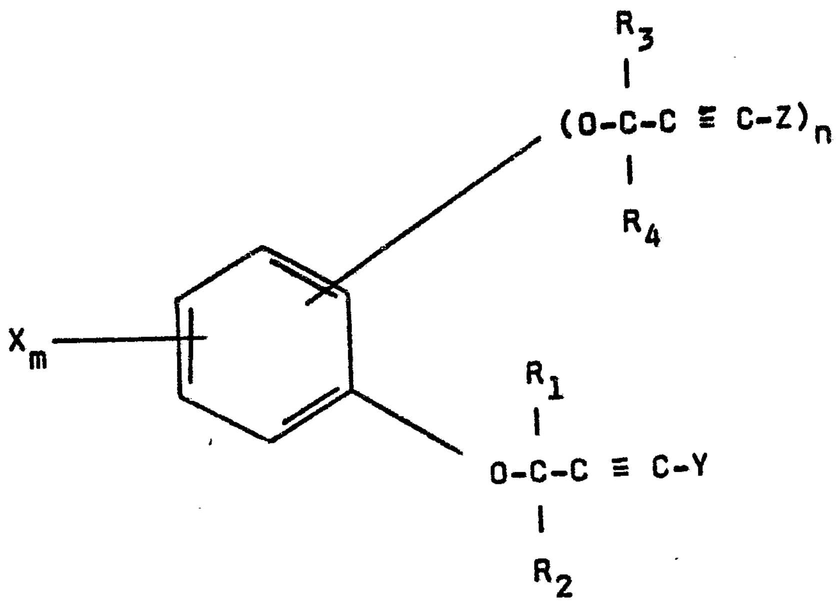

I

dans laquelle

- X est un radical identique ou différent choisi dans le groupe comprenant :
  - un atome d'hydrogène et un atome d'halogène,
  - un groupe cyano, nitro et amino éventuellement mono ou di-alcoylé,
  - un radical alcoyle, alcoxy, alcoylthio dont la partie alcoyle comprend 1 à 12 atomes de carbone et peut être mono- ou poly- halogénée, le radical alcoyle pouvant, lorsqu'il comprend de 1 à 4 atomes de carbone, être substitué par un hydroxyle, un alcoxy inférieur ou un benzoxy ou un amino éventuellement mono ou di-alcoylé,
  - alcényl ou alcénoxy de 2 à 5 atomes de carbone,
  - un phényle, phénoxy ou phénylthio éventuellement substitué,
- m est un entier égal de zéro à 5-n ;
- Y et Z identiques ou différents représentent un atome d'hydrogène ou d'halogène ;
- n est entier égal à 1 ou 2 ;

- $R_1$, $R_2$, $R_3$ et $R_4$ identiques ou différents sont chacun un atome d'hydrogène ou un radical alcoyle inférieur ; par alcoyle inférieur on entend un alcoyle contenant de 1 à 4 atomes de carbone.

Des composés préférés sont représentés par les dérivés de formule I ci-dessus avec les mêmes définitions de substituants sous réserve que,

lorsque $R_1$, $R_2$, $R_3$, $R_4$ et X sont chacun un atome d'hydrogène et n est égal à 1, les Y et Z ne sont pas tous un atome d'hydrogène.

Une sous famille préférée comprend les dérivés de formule I tels que définis ci-dessus dans la formule desquels le groupe $-O-C(R_3,R_4)-C{\equiv}C-Z$ est, sur le noyau phényle, en position ortho par rapport au groupe $-O-C(R_1, R_2)-C{\equiv}C-Y$

D'autres composés préférés sont tels que, dans la formule I, Y et Z identiques ou différents, sont un atome de brome ou d'iode.

Les composés selon l'invention sont particulièrement intéressants en raison de leurs bonnes propriétés fongicides, notamment en agriculture et peuvent être utilisés comme matière active de compositions fongicides.

Les composés utilisables selon l'invention peuvent être préparés selon des procédés en soi connus.

Les composés de formule I, dans laquelle tous les Y et Z sont un atome d'hydrogène, c'est à dire les composés I', peuvent être préparés par action en soi connue (cf Houben Weil : Methoden der Organischen Chemie Vol VI/3 p. 54 (1965)) d'un di ou triphénol sur un halogénure de propargyle, selon le schéma :

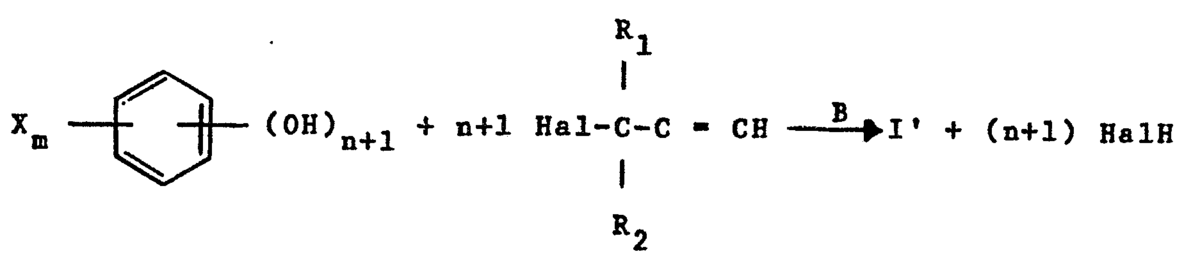

en présence d'un accepteur d'acide (B) éventuellement en milieu solvant.

Certains di et tri phénols de départ sont connus, en particulier ceux dans la formule desquels X est un atome d'hydrogène.

Comme accepteur d'acide utilisable, on peut citer les bases minérales telles que hydroxyde ou carbonate alcalins ou alcalino-terreux, en particulier la soude, la potasse, le carbonate de potassium.

On peut avantageusement effectuer la réaction en milieu solvant notamment organique tels que des cétones comme l'acétone, des nitriles comme l'acétonitrile, des amides tels que le diméthylformamide, le diméthylsulfoxyde, des éthers cycliques comme le tétrahydrofuranne, des hydrocarbures aliphatiques ou aromatiques éventuellement substitués par exemple le toluène.

Avantageusement la réaction peut être effectuée en présence de catalyseur, notamment un catalyseur de transfert de phase, tel que la tris(dioxa-3,6 heptylamine).

Les dérivés de formule I, dans laquelle au moins un des Y, Z est un atome d'halogène (= composés I") peuvent être obtenus par des méthodes en soi connues, notamment Houben Weil : Methoden der Organischen Chemie vol V/3 p. 601, (1977), par réaction d'un halogène sur un dérivé di ou tri-propargyloxybenzène selon le schéma :

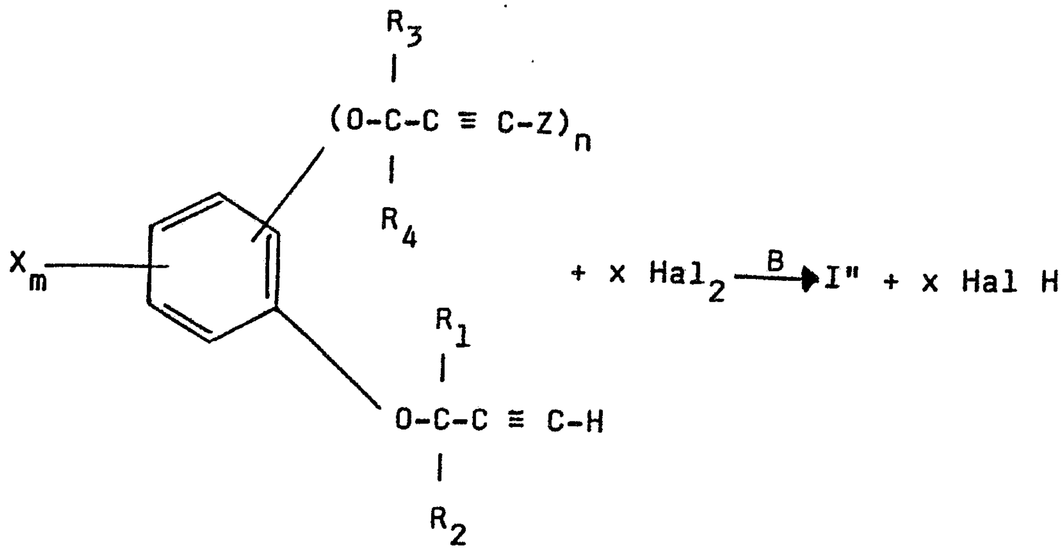

dans lequel Z représente au moins un atome d'hydrogène et Hal représente un atome d'halogène, de préférence chlore et surtout brome et iode, en présence d'un accepteur d'acide (B), éventuellement en milieu solvant.

Certains des produits de départ sont des dérivés connus, notamment quand X est un atome d'hydrogène et n est égal à 1.

Comme accepteur d'acide on peut utiliser soit des bases minérales telles que hydroxydes alcalins ou alcalino-terreux (soude, potasse, ammoniaque, carbonates de sodium, de potassium) soit des bases organiques en particulier des amines notamment amines secondaires telles que la morpholine ou encore des alcoolates alcalins comme par exemple l'éthylate ou le méthylate de sodium.

Comme solvants utilisables dans le procédé selon l'invention on peut citer, par exemple, l'eau, des alcools inférieurs (méthanol, éthanol), des éthers (diméthoxyéthane (DME)), des hydrocarbures aliphatiques ou aromatiques éventuellement halogénés ou des mélanges de ces solvants.

Les exemples suivants sont donnés à titre indicatif pour illustrer les composés selon l'invention, leur préparation et leur propriétés fongicides. La structure chimique des composés est confirmée par analyse spectrographique RMN.

Exemple 1 : Préparation du tris (propyn-2 yloxy)-1,2,3 benzène (Composé n° 22)

A une solution de 100,8 g (0,8 mole) de pyrogallol (ou trihydroxy-1,2,3 benzène) dans un mélange d'acétonitrile (350 ml) et de toluène (250 ml), on ajoute 364,3 g de carbonate de potassium (2,64 mole) et 5,2 g (0,016 mole) de tris(dioxa-3,6 heptyl) amine. On ajoute en 10 mn 285,6 g (2,4 moles) de bromure de propargyle et on chauffe 4 h à 75°C. On filtre le mélange réactionnel, lave les minéraux filtrés avec de l'acétonitrile. On lave plusieurs fois le filtrat avec de la soude diluée, puis avec de l'eau et on le concentre.

Après filtration sur silice on obtient 145 g d'huile qu'on fait cristalliser dans l'éther à - 20°C. On obtient un solide blanc fondant à 54,5°C.

Les autres composés di-(cf composés n° 1, 2, 3, 10, 11, 12, 19) et tri (cf composé n° 23) propyn-2-yl oxy-benzènes sont préparés en suivant le même mode opératoire ; leur formule et leurs caractéristiques physico-chimiques sont consignées dans le tableau I suivant.

Exemple 2 : Préparation du bis [(bromo-3 propyn-2-yl)oxy) -1,2 benzène (composé n° 4)

Dans un mélange de 288 g de soude aqueuse à 30 % et de 280 ml d'eau, on ajoute 151 g de brome, en 5mn environ, à une température comprise entre 5 et 15°C. On ajoute ensuite une solution de 50 g d'ortho di (propyn-2yl oxy) benzène dans 675 ml de diméthoxyéthane, à environ 15°C, en 15 mn environ. On agite ensuite pendant 2 heures à température ambiante. On coule le mélange réactionnel dans l'eau. Le précipité obtenu est filtré et lavé abondamment à l'eau et séché sous vide. Après recristallisation dans un

mélange d'heptane (90 vol) et d'acétate d'éthyle (10 vol), on obtient 65 g, soit avec un rendement de 70 %, d'un solide blanc fondant à 93-93,5°C. (Analyse centésimale
Calculé %C : 41,90 ; H : 2,34 ; Br : 46,46
Trouvé %C : 41,95 ; H : 2,45 ; Br : 46,30).

Exemple 3 : Préparation du bis [(iodo-3 propyn-2-yl)oxy] -1,2 benzène (composé n° 7)

A une solution de 26 g d'ortho di(propyn-2yl oxy) benzène dans 240 ml de méthanol, on ajoute simultanément et en 15 mn environ, à température ambiante, une solution de 35,8 g d'hydroxyde de sodium dans un mélange de 160 ml de méthanol, et 40 ml d'eau, et 106,7 g d'iode par petites fractions. Après réaction, pendant 2 heures environ à température ambiante, le mélange réactionnel est coulé dans une solution à 2 % de thiosulfate de sodium. Le précipité obtenu est filtré, lavé abondamment à l'eau et séché sous vide.

Après recristallisation dans le cyclohexane on obtient 51,2 g (soit avec un rendement de 83,5 %) d'un solide blanc fondant à 116 - 116,5°C (Analyse centésimale
Calculé %C : 32,91 ; H : 1,84 ; I : 57,94
Trouvé %C : 32,81 ; H : 1,90 ; I : 57,80)

Exemple 4 :

En opérant selon le même mode opératoire que celui des exemples 2 (dérivés bromés) et 3 (dérivés iodés) on obtient respectivement les composés 4 à 6, 13 à 15, 20 et 38 à 53 d'une part, et 7 à 9, 16 à 18, 21 et 54 à 65 d'autre part, dont la formule et les caractéristiques physico-chimiques (point de fusion) sont indiqués dans le tableau II suivant.

Tableau I

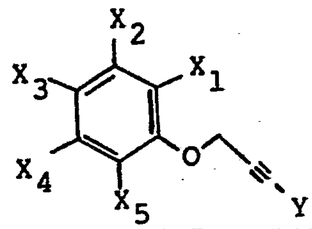

| N° | Y | $X_1$ | $X_2$ | $X_3$ | $X_4$ | $X_5$ | $0_F$ °C |
|---|---|---|---|---|---|---|---|
| 1 | H | $O-CH_2-C\equiv C-H$ | H | H | H | H | 31,5 |
| 2 | H | $O-CH_2-C\equiv C-H$ | H | $CH_3$ | H | H | 41 |
| 3 | H | $O-CH_2-C\equiv C-H$ | $OCH_3$ | H | H | H | huile |
| 10 | H | H | $O-CH_2-C\equiv C-H$ | H | H | H | 38,5 |
| 11 | H | H | $O-CH_2-C\equiv C-H$ | Cl | H | H | 55 |
| 12 | H | H | $O-CH_2-C\equiv C-H$ | Cl | H | Cl | 122 |
| 19 | H | H | H | $-O-CH_2-C\equiv C-H$ | H | H | 46,5 |
| 22 | H | $O-CH_2-C\equiv C-H$ | $O-CH_2-C\equiv C-H$ | H | H | H | 54,5 |
| 23 | H | H | $-O-CH_2-C\equiv C-H$ | H | * | H | 70 |
| 24 | H | $O-CH_2-C\equiv C-H$ | H | $C(CH_3)_3$ | H | H | 26 |
| 25 | H | $O-CH_2-C\equiv C-H$ | H | $nC_9H_{19}$ | H | H | huile |
| 26 | H | $O-CH_2-C\equiv C-H$ | H | $NO_2$ | H | H | 107,5 |
| 27 | H | $O-CH_2-C\equiv C-H$ | H | Br | H | H | 82 |
| 28 | H | $O-CH_2-C\equiv CH$ | H | CN | H | H | 95,5 |
| 29 | H | $O-CH_2-C\equiv CH$ | H | $-CH_3O-$ | H | H | 77 |
| 30 | H | $O-CH_2-C\equiv CH$ | H | $C_2H_5O$ | H | H | 110,5 |
| 31 | H | $O-CH_2-C\equiv CH$ | H | $CH_2=CH-CH_2O$ | H | H | 81 |
| 32 | H | $O-CH_2-C\equiv CH$ | H | phényl-O | H | H | 72 |

| | | | | | | | |
|---|---|---|---|---|---|---|---|
| 33 | H | $O-CH_2-C\equiv CH$ | $CH_2=C(CH_3)-CH_2-$ (H) | $nC_5H_{11}-O-$ | H | H | 52,5 |
| 34 | H | $OCH_2-C\equiv C-H$ | $CH_2=C(CH_3)-CH_2-$ | H | H | H | huile |
| 35 | H | $OCH_2-C\equiv C-H$ | $C_2H_5O$ | H | H | H | 31,5 |
| 36 | H | $OCH_2-C\equiv CH$ | $C(CH_3)_3$ | H | ** | H | 56 |
| 37 | H | $OCH_2-C\equiv CH$ | H | Br | Br | H | 97 |

* = $O-CH_2-C\equiv C-H$

** = $C(CH_3)_3$

Tableau II

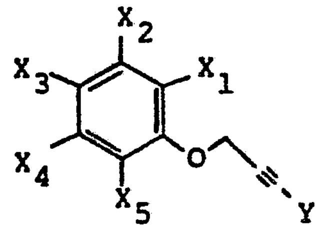

| N° | Y | $X_1$ | $X_2$ | $X_3$ | $X_4$ | $X_5$ | $0_F$ °C |
|---|---|---|---|---|---|---|---|
| 4 | Br | $O-CH_2-C≡C-Br$ | H | H | H | H | 93-93,5 |
| 5 | Br | $O-CH_2-C≡C-Br$ | H | $CH_3$ | H | H | 92-92,5 |
| 6 | Br | $O-CH_2-C≡C-Br$ | $OCH_3$ | H | H | H | 76,5 |
| 7 | I | $O-CH_2-C≡C-I$ | H | H | H | H | 116-116,5 |
| 8 | I | $O-CH_2-C≡C-I$ | H | $CH_3$ | H | H | 98,5 |
| 9 | I | $O-CH_2-C≡C-I$ | $OCH_3$ | H | H | H | 101 |
| 13 | Br | H | $O-CH_2-C≡C-Br$ | H | H | H | 85,5 |
| 14 | Br | H | $O-CH_2-C≡C-Br$ | Cl | H | H | 77,5 |
| 15 | Br | H | $O-CH_2-C≡C-Br$ | Cl | H | Cl | 136 |
| 16 | I | H | $O-CH_2-C≡C-I$ | H | H | H | 111 |
| 17 | I | H | $O-CH_2-C≡C-I$ | Cl | H | H | 116,5 |
| 18 | I | H | $O-CH_2-C≡C-I$ | Cl | H | Cl | 155 |
| 20 | Br | H | H | $O-CH_2-C=C-Br$ | H | H | 100,5 |
| 21 | I | H | H | $O-CH_2-C=C-I$ | H | H | 120,5 |
| 38 | Br | $OCH_2-C≡C-Br$ | $OCH_3$ | H | *** | H | 87,5 |
| 39 | Br | $OCH_2-C≡C-Br$ | H | Br | Br | H | 112,5 |
| 40 | Br | $OCH_2-C≡C-Br$ | $OCH_2-C≡C-Br$ | H | H | H | 90,5 |

| | | | | | | | |
|---|---|---|---|---|---|---|---|
| 41 | Br | $O-CH_2-C\equiv C-Br$ | $CH_2=C(CH_3)-CH_2$ | H | H | H | huile |
| 42 | Br | $O-CH_2-C\equiv C-Br$ | $C_2H_5O$ | H | H | H | 105,5 |
| 43 | Br | $O-CH_2-C\equiv C-Br$ | H | $C(CH_3)_3$ | H | H | 64 |
| 44 | Br | $O-CH_2-C\equiv C-Br$ | H | $C_9H_{19}$ | H | H | huile |
| 45 | Br | $O-CH_2-C\equiv C-Br$ | H | $NO_2$ | H | H | 145,5 |
| 46 | Br | $O-CH_2-C\equiv C-Br$ | H | Br | H | H | 115,5 |
| 47 | Br | $O-CH_2-C\equiv C-Br$ | H | CN | H | H | 119 |
| 48 | Br | $O-CH_2-C\equiv C-Br$ | H | $HOCH_2$ | H | H | 92 |
| 49 | Br | $O-CH_2-C\equiv C-Br$ | H | $CH_3O$ | H | H | 90 |
| 50 | Br | $O-CH_2-C\equiv C-Br$ | H | $C_2H_5O$ | H | H | 102,5 |
| 51 | Br | $O-CH_2-C\equiv C-Br$ | H | $CH_2=CH-CH_2O$ | H | H | 110,5 |
| 52 | Br | $O-CH_2-C\equiv C-Br$ | H | $C_6H_5-O$ | H | H | 63 |
| 53 | Br | $O-CH_2-C\equiv C-Br$ | H | $nC_5H_{11}O$ | H | H | 59 |
| 54 | I | $OCH_2-C\equiv I$ | H | $C(CH_3)_3$ | H | H | 118 |
| 55 | I | $OCH_2-C\equiv I$ | H | $C_9H_{19}$ | H | H | huile |
| 56 | I | $OCH_2-C\equiv I$ | H | $NO_2$ | H | H | 154,5 |
| 57 | I | $OCH_2-C\equiv I$ | H | Br | H | H | 136,5 |
| 58 | I | $OCH_2-C\equiv I$ | H | CN | H | H | 158 |
| 59 | I | $OCH_2-C\equiv I$ | H | $OCH_3$ | H | H | 107 |
| 60 | I | $OCH_2-C\equiv I$ | H | $OC_2H_5$ | H | H | 129 |
| 61 | I | $OCH_2-C\equiv I$ | H | $CH_2=CH-CH_2O$ | H | H | 132,5 |
| 62 | I | $OCH_2-C\equiv I$ | H | $C_6H_5-O$ | H | H | miel |
| 63 | I | $OCH_2-C\equiv I$ | H | $nC_5H_{11}-O$ | H | H | 83,5 |
| 64 | I | $OCH_2-C\equiv I$ | $CH_2=C(CH_3)-CH_2$ | H | H | H | huile |
| 65 | I | $OCH_2-C\equiv I$ | $C_2H_5O$ | H | H | H | 117 |

*** = $HOCH_2$

Exemple 5 : Tests in vitro sur champignons des semences et champignons du sol

On étudie l'action des composés selon l'invention sur les champignons suivants :

- Botrytis cinerea : souche résistante à la fois au carbendazime et à l'iprodione
- Pseudocercosporella herpotrichoïdes : souche résistante au carbendazime
- Pythium aphanidermatum
- Phytophtora cinnamomi
- Rhizoctonia solani

Pour chaque essai, on opère de la manière suivante: un milieu nutritif constitué de pomme de terre, de glucose et de gelose (milieu PDA) est introduit en surfusion dans une série de boîtes de Pétri (20 ml par boîte) après stérilisation à l'autoclave à 120°C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycelium provenant d'une culture précédente du même champignon.

Les boîtes sont conservées pendant 2 à 10 jours (selon le champignon testé) à 22°C et on compare alors la croissance du champignon dans les boîtes contenant la matière active à tester, à celle du même champignon dans la boîte utilisée comme témoin.

Dans ces conditions, on observe une protection bonne ou totale (c'est à dire au moins égale à 80 %) :

- sur Botrytis cinerea, à la dose de 0,03 g/l pour les composés 2 à 9, 11 à 19, 21, 22, 24 à 35, 37, 38, 40, 42, 46, 47, 49 à 51, 57 à 60, 64 et 65,
- sur Pseudocercosporella herpotrichoïdes, à la dose de 0,03 g/l pour les composés 2, 7 à 11, 16, 17, 19, 21 et 22, 24 à 35, 37 à 40, 42, 46, 47, 49 à 51, 57 à 60 et 65,
- sur Pythium aphanidermatum et Phytophthora cinnamomi, à la dose de 0,1 g/l, pour les composés 2, 3, 7, 8, 10, 11, 13, 16, 17, 19, 21 et 22,
- sur Rhizoctonia solani, à la dose de 0,1 g/l pour les composés 2, 5, 7, 8, 10, 11, 16, 17, 19 et 21.

Exemple 6 : Test sur Botrytis cinerea sur tomate :

Des tomates cultivées en serre (variété Marmande) agées de 30 à 40 jours sont traitées par pulvérisation avec des suspensions aqueuses (appelées bouillies) ayant la composition suivante :

- matière active micronisée ............... x mg
- condensat d'oxyde d'éthylène (20 moles) de monooléate de sorbitan .............. x/2 mg
- eau qsp ................................ 60 ml

et à diverses concentrations du composé à tester. L'essai est répété deux fois avec chaque concentration.

Après 24 heures, les feuilles sont coupées et mises dans 2 boîtes de Pétri (diamètre 14 cm) dont le fond a été préalablement garni d'un disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea, souche résistante aux benzimidazoles, a été obtenue à partir d'une culture de 15 jours, mise ensuite en suspension dans une solution nutritive (100.000 unités/$cm^3$).

Le contrôle est fait 6 jours après la contamination par comparaison avec un témoin non traité.

Dans ces conditions on observe, à la dose de 0,1 g/l, une protection bonne ou totale (c'est à dire au moins égale à 80 %) avec les composés n° 4, 7, 13, 16 et 22.

Exemple 7 : Expérimentation en serre sur mildiou de la vigne (Plasmopara viticola)

Des boutures de vigne (vitis vinifera), de variété Chardonnay, sont cultivées dans des godets. Lorsque ces plants sont âgés de 2 mois (stade 8 à 10 feuilles, hauteur 20 à 30 cm), ils sont traités par pulvérisation au moyen d'une suspension ou solution aqueuse de la matière à tester, telle que décrite à l'exemple 6. Chaque plant de vigne reçoit environ 5 ml de la solution ou dispersion. Pour chaque concentration de matière active à tester, le traitement est effectué sur deux plants. Des plants utilisés comme témoins sont traités par une solution ne contenant pas de matière active, mais contenant une concentration identique du même condensat de monooléate de sorbitan et d'oxyde d'éthylène.

Après séchage pendant 24 heures, on contamine chaque plant par pulvérisation au moyen d'une suspension aqueuse de spores de Plasmopara Viticola, responsable du mildiou de la vigne, à raison d'environ 1 ml/plant (soit environ $10^5$ spores par plant).

Après cette contamination, les plants de vigne sont mis en incubation pendant deux jours à 18°C environ en atmosphère saturée d'humidité, puis pendant cinq jours à 20-22°C environ sous 90-100 % d'humidité relative.

Sept jours après la contamination, on compare les résultats obtenus dans le cas des plants traités par la matière active à tester à ceux obtenus dans le cas des plants utilisés comme témoin.

Dans ces conditions, on a observé que, à la dose de 1 g/l, les composés n° 7 et 16 entraînent 100 % d'inhibition du développement du champignon.

Les doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

Selon ce qui à déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc..., ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %),

BAD ORIGINAL

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les granulés, les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses selon l'invention :

Exemple A :

On prépare une suspension aqueuse comprenant :

- matière active (composé n° 4)................ 500 g/l
- agent mouillant (alcool synthétique en $C_{13}$ polyéthoxylé) .......................... 10 g/l
- agent dispersant (phosphate de polyaryl phénol éthoxylé salifié) ......................... 50 g/l
- antigel (propylèneglycol) ................. 100 g/l
- épaississant (polysacharide) ............... 1,6 g/l
- biocide (méthylhydroxy-4 benzoate sodé) .... 3,3 g/l
- eau ................................Q.S.P.1 litre.

On obtient ainsi une suspension concentrée fluide.

Exemple B - Suspension aqueuse :

On prépare une suspension aqueuse comprenant :

- matière active (composé n° 7)............... 100 g/l
- agent mouillant (alkylphénol polyéthoxylé .. 5 g/l
- agent dispersant (Naphtalène sulfonate de Na 10 g/l
- antigel (Propylèneglycol) .................. 100 g/l
- épaississant (Polysacharide) .............. 3 g/l
- biocide (Formaldéhyde) ..................... 1 g/l
- eau ................................Q.S.P.1 litre.

Exemple C - Suspension aqueuse :

On prépare une suspension aqueuse comprenant :

- matière active (composé n° 22)............... 250 g/l
- agent mouillant (alcool synthétique en $C_{13}$ polyéthoxylé ........................... 10 g/l
- agent dispersant (lignosulfonate de solium) 15 g/l
- antigel (urée) ........................... 50 g/l
- épaississant (Polysacharide) .............. 2,5 g/l
- biocide (Formaldéhyde) ..................... 1 g/l
- eau ................................Q.S.P.1 litre.

Les poudres mouillables (ou poudre à pulvériser) sont habituellement préparées de manière qu'elles contiennent 20 à 95 % de matière active, et elles

contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici la composition de plusieurs poudres mouillables.

- matière active (composé n° 1 selon l'invention) 50 %
- alcool gras éthoxylé (agent mouillant).......... 2,5 %
- styrylphénol éthoxylé (agent dispersant)........ 5 %
- craie (support inerte) ..........................42,5 %

<u>Exemple E : Poudre mouillable à 10 %</u>

- matière active (composé n° 4) ...................10 %
- alcool synthétique oxo de type ramifié, en $C_{13}$ éthoxylé par 8 à 10 oxyde d'éthylène (agent mouillant) ..........................................0,75 %
- lignosulfonate de calcium neutre (agent dispersant ........................................ 12 %
- carbonate de calcium (charge inerte) .......qsp 100 %

<u>Exemple F : Poudre mouillable à 75 %</u> contenant les mêmes ingrédients que dans l'exemple précédent, dans les proportions ci-après :

- matière active (composé n° 7) ................... 75 %
- agent mouillant .................................1,50 %
- agent dispersant .................................8 %
- carbonate de calcium (charge inerte) .......qsp 100 %

<u>Exemple G : Poudre mouillable à 90 %</u>

- matière active (composé n° 22) ..................90 %
- alcool gras éthoxylé (agent mouillant ........... 4 %
- styrylphénol éthoxylé (agent dispersant) ........ 6 %

Exemple H : Poudre mouillable à 50 %

- matière active (composé n° 4 selon l'invention)..50 %
- mélange de tensio-actifs anioniques et non ioniques (agent mouillant) ......................2,5 %
- lignosulfonate de sodium neutre (agent dispersant) ....................................... 5 %
- argile kaolinique (support inerte) .............42,5 %

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6, ont une dimension de particules généralement comprise entre environ 150 et 2000 et de préférence entre 300 et 1500 microns.

La teneur en matière active (composé n° 4) de ces granulés est généralement comprise entre environ 1 % et 90 %, et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge retenue est soluble ou non dans l'eau. Lorsque la charge est hydrosoluble, elle peut être minérale et de préférence organique. On a obtenu d'excellents résultats avec l'urée.

Dans le cas d'une charge insoluble, celle-ci est de préférence minérale, comme par exemple le kaolin ou la bentonite. Elle est alors accompagnée d'agents tensio-actifs (à raison de 2 à 20 % en poids du granulé), adjuvants tensio-actifs dont plus de la moitié est constituée par au moins un agent dispersant, essentiellement anionique, tel qu'un poly(naphtalène sulfonate alcalin ou alcalino terreux) ou un lignosulfonate alcalin ou alcalino-terreux, le reste étant constitué par des mouillants non ioniques ou anioniques tel qu'un alcoyl naphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc..). On termine généralement par un concassage suivi d'un tamisage à la dimension de particule choisie dans les limites mentionnées ci-dessus.

De préférence, il est obtenu par extrusion, en opérant comme indiqué dans les exemples ci-après.

<u>Exemple I : Granulés dispersibles à 90 % de matière active</u>

Dans un mélangeur, on mélange 90 % en poids de matière active (composé n° 7) et 10 % d'urée en perles. Le mélange est ensuite broyé dans un broyeur à broches. On obtient une poudre que l'on humidifie avec environ 8 % en poids d'eau. La poudre humide est extrudée dans une extrudeuse à rouleau perforé. On obtient un granulé qui est séché, puis concassé et tamisé, de façon à ne garder respectivement que les granulés d'une dimension comprise entre 150 et 2000 microns.

Exemple J : Granulés dispersibles à 75 % de matière active

Dans un mélangeur, on mélange les constituants suivants :

- matière active (composé n° 22) .................. 75 %
- agent mouillant (alkylnaphtalène sulfonate de sodium ......................................... 2 %
- agent dispersant (polynaphtalène sulfonate de sodium) ........................................ 8 %
- charge inerte insoluble dans l'eau (kaolin) ..... 15 %

Ce mélange est granulé en lit fluide, en présence d'eau, puis séché, concassé et tamisé de manière à obtenir des granulés de dimension comprise entre 0,16 et 0,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

L'invention concerne de plus un procédé de traitement des végétaux contre les maladies causées par les champignons phytopathogènes appartenant aux famille ples plus variées et notamment sur les Botrytis sp souches sensibles ou résistantes aux benzimidazoles, le piétin verse (souches sensibles ou résistantes eux benzimidazoles), les champignons des semenses tels que Pythium sp, Fusarium sp, Septoria sp, Rhizoctonia sp ou

encore les champignons responsables des pourritures tels que Monilia sp, Penicillium sp, Rhizopus sp ou encore Venturia sp, Phytophthora sp. etc...

Ce procédé est caractérisé en ce qu'il consiste à appliquer sur ces végétaux une quantité efficace d'une composition contenant comme matière active un composé selon la formule (I). Par "quantité efficace" on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 1 g/hl à 500 g/hl correspondant sensiblement à des doses de matière active par hectare de 10 g/ha à 5000 g/ha environ donnent généralement de bons résultats.

## REVENDICATIONS

1) Dérivés du propargyloxybenzène de formule :

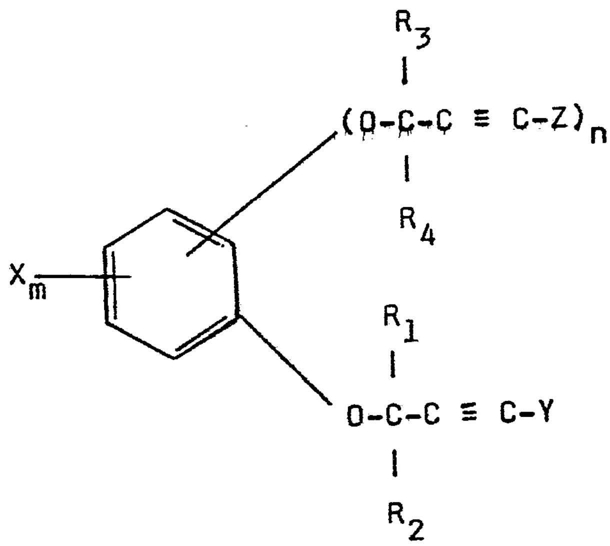

I

dans laquelle

- X est un radical identique ou différent choisi dans le groupe comprenant :
  - un atome d'hydrogène et un atome d'halogène,
  - un groupe cyano, nitro et amino éventuellement mono ou di-alcoyle,
  - un radical alcoyle, alcoxy, alcoylthio dont la partie alcoyle comprend 1 à 12 atomes de carbone et peut être mono- ou poly- halogénée, le radical alcoyle pouvant, lorsqu'il comprend de 1 à 4 atomes de carbone, être substitué par un hydroxyle, un alcoxy inférieur ou un benzoxy ou un amino éventuellement mono ou di-alcoylé,
  - alcényl ou alcénoxy de 2 à 5 atomes de carbone,
  - un phényle, phénoxy ou phénylthio éventuellement substitué,
- m est un entier égal de zéro à 5-n ;
- Y et Z identiques ou différents représentent un atome d'hydrogène ou d'halogène ;
- n est entier égal à 1 ou 2 ;

- $R_1$, $R_2$, $R_3$ et $R_4$ identiques ou différents sont chacun un atome d'hydrogène ou un radical alcoyle inférieur, sous réserve que, lorsque $R_1$, $R_2$, $R_3$, $R_4$ et X sont chacun un atome d'hydrogène et n est égal à 1, les Y et Z ne sont pas tous un atome d'hydrogène.

2) Dérivés selon la revendication 1, caractérisés en ce que n est égal à 1 et le groupe $-O-C(R_3, R_4)-C \equiv C-Z$ est, sur le noyau phényle, en position ortho par rapport au groupe $-O-C(R_1, R_2)-C \equiv C-Y$.

3) Dérivés selon la revendication 1, caractérisés en ce que Y et Z identiques ou différents sont un atome de brome ou d'iode.

4) Procédé de préparation des composés selon les revendications 1 à 3 dans la formule desquels les X ne sont pas tous un atome d'hydrogène lorsque n est égal à 1 et les Y et Z sont un atome d'hydrogène, caractérisé en ce qu'on fait réagir un di ou triphénol sur un halogénure de propargyle selon le schéma :

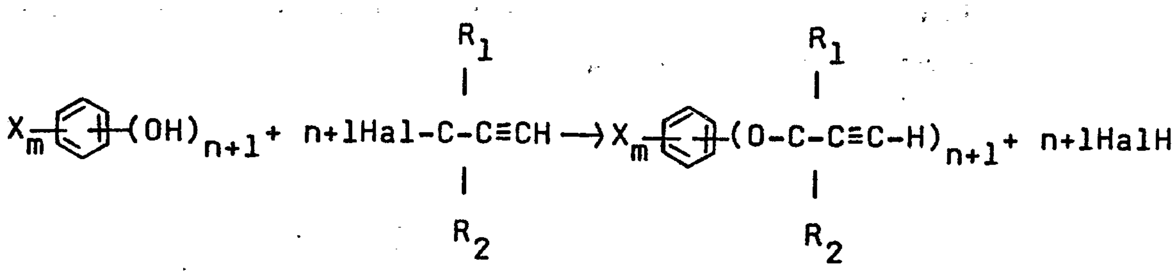

en présence d'un accepteur d'acide.

5) Procédé de préparation des composés selon les revendications 1 à 3 dans la formule desquels au moins un Y ou Z est un atome d'halogène, caractérisé en ce qu'on effectue la réaction suivante :

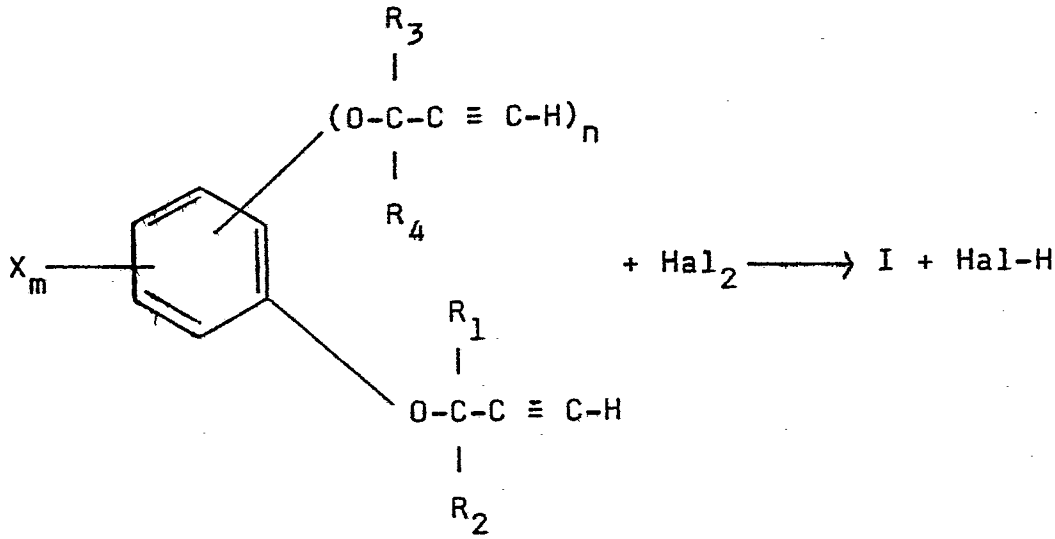


dans laquelle Hal est un atome d'halogène, en présence d'un accepteur d'acide,

6) Procédé selon la revendication 5, caractérisé en ce que dans le schéma Hal est un atome d'iode ou de brome.

7) Compositions fongicides pour la lutte contre les maladies fongiques des plantes caractérisées en ce qu'on applique une composition contenant comme matière active un composé de formule ;

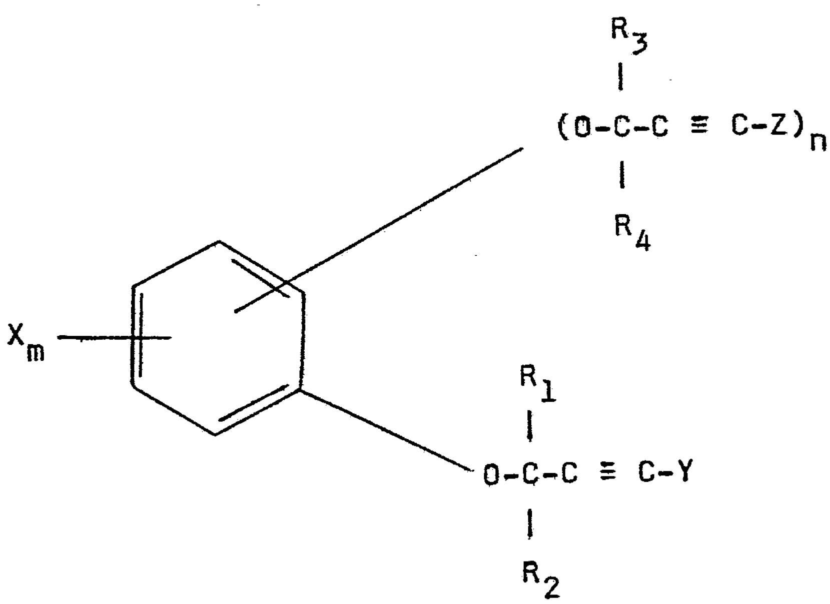

I

dans laquelle

- X est un radical identique ou différent choisi dans le groupe comprenant :
  - un atome d'hydrogène et un atome d'halogène,
  - un groupe cyano, nitro et amino éventuellement mono- ou di-alcoylé,
  - un radical alcoyle, alcoxy, alcoylthio dont la partie alcoyle comprend 1 à 12 atomes de carbone et peut être mono- ou poly- halogénée, le radical alcoyle pouvant, lorsqu'il comprend de 1 à 4 atomes de carbone, être substitué par un hydroxyle, un alcoxy inférieur ou un benzoxy ou un amino éventuellement mono ou di-alcoylé,
  - alcényl ou alcénoxy de 2 à 5 atomes de carbone,
  - un phényle ou phénoxy éventuellement substitué,
- m est un entier égal de zéro à 5-n ;
- Y et Z identiques ou différents représentent un atome d'hydrogène ou d'halogène ;
- n est entier égal à 1 ou 2 ;
- $R_1$, $R_2$, $R_3$ et $R_4$ identiques ou différents sont chacun un atome d'hydrogène ou un radical alcoyle inférieur.

8) Composition selon la revendication 7 caractérisée en ce que les substituants ont la même signification sous réserve que, lorsque $R_1$, $R_2$, $R_3$, $R_4$ et X sont chacun un atome d'hydrogène et n est égal à 1, les Y et Z ne sont pas tous un atome d'hydrogène.

9) Procédé de lutte contre les maladies fongiques des plantes caractérisé en ce qu'on applique à ces dernières une composition contenant comme matière active un composé selon l'une des revendications 7 et 8.